# EUROPEAN PATENT APPLICATION

(11) **EP 3 868 891 A1**
(43) Date of publication of application: **25.08.2021**
(21) Application number: 19874165.4
(22) Date of filing: 18.10.2019
(51) Int. Cl.: C12Q 1/02, G01N 1/02, G01N 1/10

(54) **METHOD FOR EXTRACTING PARTICULATE MATTER IN SMOKE FROM HEATED SMOKING ARTICLE, EXTRACTION CONTAINER, EXTRACTION LIQUID, AND TOXICITY TESTING METHOD USING EXTRACTION LIQUID**

(30) Priority: 19.10.2018 JP 2018197672
(71) Applicant: Japan Tobacco Inc., Tokyo 105-6927 (JP)
(72) Inventor: ITO, Hiroshi, Tokyo 105-8422 (JP); KATO, Tadashi, Tokyo 105-0021 (JP); TSUNODA, Shinya, Tokyo 105-0021 (JP)
(74) Representative: Hoffmann Eitle
(86) International application number: PCT/JP2019/041101
(87) International publication number: WO 2020/080529

(57) **Abstract**

An object of the present invention is to provide a method for extracting particulate matter from the smoke of a heated smoking article, an extraction container, an extract liquid, and a toxicity test method involving use of the extract. The method of the present invention comprises: (1) collecting particulate matter in the smoke of a heated smoking article in two or more filters capable of capturing the particulate matter; (2) stacking the two or more filters; and (3) squeezing the stacked filters to extract components adhered to the filters. An extraction container 1a of the present invention comprises: an accommodation area 8 capable of accommodating two or more filters 7 in a stacked state; one or more squeezing members (2a, 3a) for squeezing the filters 7 accommodated in the accommodation area 8 in a stacked state; and a reservoir 10 communicating with the accommodation area 8.

## Description

### TECHNICAL FIELD

The present invention relates to a method for extracting particulate matter from the smoke of a smoking article, which is carried out by a person skilled in the fields of toxicity science among the fields of tobacco manufacturing industry, and in particular a method for extracting particulate matter from the smoke of a heated smoking article, and an extraction container, an extract liquid, and a toxicity test method using the extract liquid.

### BACKGROUND ART

Combustible smoking articles, such as cigarettes, typically include shredded tobacco (usually in the form of cut filler) surrounded by a paper wrapper that forms a tobacco rod. A consumer uses each cigarette by lighting one end of the cigarette and burning the shredded tobacco rod. The consumer inhales the smoke from the opposite end (the end on the mouth side or the filter end) of the burnt cigarette to receive the mainstream smoke.

On the other hand, various "heated smoking articles" have been proposed, in which the nicotine-containing aerosol-forming substrate, such as tobacco, is of a heated-type instead of a combustion type. In heated smoking articles, aerosols are produced by heating an aerosol-forming substrate. Known heated smoking articles include, for example, smoking articles in which aerosols are electrically heated or produced by transfer of heat from a combustible fuel element or a heat source to an aerosol-forming substrate. Furthermore, in another type, a porous or fibrous substance impregnated with alcohol, ethanol, glycerin, propylene glycol, or a mixed solution thereof is directly or indirectly heated with an electric heat source or a heat source based on chemical reactions, so as to generate vapor. In this type, the gas in which the generated vapor and the particulate matter are mixed is mixed in the air sucked through a smoking article, and the gaseous mixture of the vapor, the particulate matter and the air passes through the above-mentioned tobacco, or tobacco leaf, chopped tobacco, reconstituted tobacco, reconstituted tobacco sheet, or reconstituted tobacco granules. During this passage, the gaseous mixture is mixed with a gas containing a smoking flavor component contained in tobacco, tobacco leaf, chopped tobacco, and reconstituted tobaccos, and volatile compounds including nicotine or various natural flavors. In the heated smoking article of this type, a consumer inhales the gaseous mixture containing the volatile compound-containing gas, that is, the gaseous mixture of aerosols (particle phase component) and the gas phase components. Typically, a consumer sucks at an end (the end on the mouth side, or the filter end or the mouthpiece end) of the heated smoking article to inhale the gaseous mixture.

The smoke generated from smoking articles such as cigarettes contains substances including tar and nicotine. Regarding smoking articles, it is important to capture particulate matter in smoke components, for example, on a carrier such as a filter to analyze and evaluate the components. For example, a method shown by the Health Canada is known as a standard for collecting and analyzing smoke components of cigarettes (Health Canada-Tobacco Reporting Regulations SOR/2000-273, July 2018) (NPL 1).

The amount of the particulate matter in smoke components of heated smoking articles is generally less than that of combustible smoking articles such as cigarettes, and the particulate matter in smoke components of heated smoking articles cannot be captured in a sufficient amount and at a sufficient concentration. Therefore, it is difficult for those skilled in the tobacco manufacturing industry who are in charge of analysis of smoke components, particularly those skilled in the field of toxicity science, to obtain appropriate toxicity test results conventionally or publicly required.

Regarding also heated smoking articles, it is important that those skilled in the tobacco manufacturing industry who are in charge of analysis of smoke components, especially those skilled in the field of toxicity science, capture particulate matter in a sufficient amount and at a sufficient concentration to thereby prepare a sample in order to obtain appropriate toxicity test results conventionally or publicly required. A possible method is a continuous extraction method that involves allowing a carrier for capturing to continuously capture smoke components generated from a large amount of heated smoking articles, followed by extraction. For example, one filter is designed to capture smoke components of 100 smoking articles in this method. This enables increase in the amount of particulate matter captured from only about 10 mg/mL to a concentration as high as about 50 to 100 mg/mL, for example. However, this method takes an enormous amount of time, and the possibility of volatilizing components from the particulate matter captured during that time cannot be ruled out. Furthermore, an error may occur in overestimation due to enrichment, because of the use of a large amount of heated smoking articles. Another possible method is a method using a large amount of a solvent. However, as a higher concentration of the particulate matter is prepared in the method using a large amount of a solvent, the solvent is to be concentrated to a high concentration, and this is also the case even with a low-toxicity solvent. For example, dimethyl sulfoxide (DMSO) may be used to capture tobacco smoke components. However, DMSO also has weak cytotoxicity. In a toxicity test *(in vitro* toxicity test) using cultured cells of prokaryotes or eukaryotes, DMSO contained at a high concentration in the medium affects the physiological activity of the cells. When an *in vitro* toxicity test is performed, the upper limit of DMSO that can be used as a solvent is specified to be about 2% in terms of volume percentage with respect to the medium (NPLs 2 and 3). If an *in vitro* toxicity test is performed using captured particulate matter in a medium containing DMSO at concentration higher than the specified concentrations, the physiological activity of cells may decrease due to DMSO regardless of the particulate matter, and the cells may die, for example; thus, effective toxicity tests cannot be performed properly.

### CITATION LIST

### PATENT LITERATURE

PTL 1: Japanese Patent Laid-Open No. 2018-57403

### NON PATENT LITERATURE

NPL 1: Health Canada-Tobacco Reporting Regulations SOR/2000-273, July 2018
NPL 2: "Bacterial Reverse Mutation Test" of "OECD Guidelines for the Testing of Chemicals" (OECD/OCDE TG471) (adopted July 21, 1997)
NPL 3: "In Vitro Mammalian Cell Micronucleus Test" of "OECD Guidelines for the Testing of Chemicals" (OECD/OCDE TG 487) (adopted September 26, 2014)
NPL 4: CORESTA RECOMMENDED METHOD No. 81 ROUTINE ANALYTICAL MACHINE FOR E-CIGARETTE AEROSOL GENERATION AND COLLECTION-DEFINITIONS AND STANDARD CONDITIONS (June 2015)
NPL 5: ISO 3402
NPL 6: ISO 3308
NPL 7: ISO4387
NPL 8: Health Canada Official Method T-501 Bacterial Reverse Mutation Assay for Mainstream Tobacco Smoke
NPL 9: CORESTA Recommended Method No. 84 DETERMINATION OF GLYCERIN, PROPYLENE GLYCOL, WATER, AND NICOTINE IN THE AEROSOL OF E-CIGARETTES BY GAS CHROMATOGRAPHIC ANALYSIS
NPL 10: Matsushima et al., 1999, Environ. Pollut. 64, 121-132
NPL 11: Health Canada Official Method T-502,Neutral Red Uptake Assay for Mainstream Tobacco Smoke (Health Canada, 2004c)

### SUMMARY OF INVENTION

### TECHNICAL PROBLEM

An object of the present invention is to provide a method for extracting particulate matter from the smoke of a heated smoking article.

Another object of the present invention is to provide an extraction container. The extraction container can be used in a method for extracting particulate matter from the smoke of a heated smoking article.

Still another object of the present invention is to provide an extract containing particulate matter in the smoke of a heated smoking article.

Yet another object of the present invention relates to an *in vitro* toxicity test method involving use of an extract liquid containing particulate matter in the smoke of a heated smoking article.

### SOLUTION TO PROBLEM

As a result of intensive studies, the inventors of the present invention have found that components captured by filters can be extracted with high efficiency by stacking two or more filters that have captured particulate matter and squeezing the stacked filters. Based on this finding, the inventors have conceived of the present invention.

The present invention includes, but is not limited to, the following embodiments.
[Embodiment 1]
   A method for extracting particulate matter from the smoke of a heated smoking article, comprising:
   (1) collecting particulate matter in the smoke of a heated smoking article in two or more filters capable of capturing the particulate matter,
   (2) stacking the two or more filters, and
   (3) squeezing the stacked filters to extract components adhered to the filters.
[Embodiment 2]
   The method according to embodiment 1, wherein step (3) is performed by
   (3-i) compressing and releasing the stacked filters,
   (3-ii) compressing the stacked filters to extract components adhered to the filters, pouring the extract liquid to the stacked filters, and then compressing the filters again, or
   (3-iii) performing (3-i) and (3-ii) in combination.
[Embodiment 3]
   The method according to embodiment 2, wherein step (3-i) of compressing and releasing the stacked filters is repeated two or more times.
[Embodiment 4]
   The method according to embodiment 2, wherein the filters and the extract liquid are shaken together during steps (3-i) and/or (3-ii).
[Embodiment 5]
   The method according to embodiment 2, wherein step (3-ii) of compressing the stacked filters to extract components adhered to the filters, pouring back the extract liquid to the stacked filters, and then compressing the filters again is repeated two or more times.
[Embodiment 6]
   The method according to any one of embodiments 1 to 5, wherein step (3) is performed on the filters accommodated in an extraction container having two squeezing surfaces.
[Embodiment 7]
   The method according to any one of embodiments 1 to 6, wherein in step (3), the extract liquid obtained by squeezing is made to flow or circulate.
[Embodiment 8]
   The method according to any one of embodiments 1 to 7, wherein three or more filters are used.
[Embodiment 9]
   The method according to any one of embodiments 1 to 8, wherein 10 to 15 filters are used.
[Embodiment 10]
   The method according to any one of embodiments 1 to 9, wherein step (3) is repeated until the thickness of the stacked filters becomes 1/3 or less than the thickness before performing step (3).
[Embodiment 11]
   The method according to any one of embodiments 1 to 10, wherein no solvent is used in step (3).
[Embodiment 12]
   The method according to any one of embodiments 1 to 10, wherein in step (3), an amphipathic solvent is added in an amount of more than 0% (weight/volume) and 400% (weight/volume) or less with respect to the filter volume.
[Embodiment 13]
   The method according to any one of embodiments 1 to 12, wherein compression is performed at 10 MPa or less.
[Embodiment 14]
   The method according to any one of embodiments 1 to 13, wherein the filters are glass fiber filters.
[Embodiment 15]
   The method according to any one of embodiments 1 to 14, wherein the filters have a particle collection efficiency of 99% or more for collecting particles having a particle size of 0.3 µm at a rated air volume.
[Embodiment 16]
   The method according to any one of embodiments 1 to 15, further comprising
   (4) centrifuging the filters to obtain a separated liquid; and
   (5) mixing the separated liquid with the extract liquid obtained in step (3).
[Embodiment 17]
   An extraction container, comprising
   an accommodation area capable of accommodating two or more filters in a stacked state,
   one or more squeezing members for squeezing the filters accommodated in the accommodation area in a stacked state; and
   a reservoir communicating with the accommodation area.
[Embodiment 18]
   The extraction container according to embodiment 17, wherein the shape of a squeezing surface for squeezing the filters with the squeezing member is a flat surface, a conical slope, or a convex or concave curved surface.
[Embodiment 19]
   The extraction container according to embodiment 17 or 18, wherein there are two squeezing surfaces for squeezing the filters with the squeezing member.
[Embodiment 20]
   The extraction container according to embodiment 19, wherein the two squeezing surfaces rotate and slide relative to each other.
[Embodiment 21]
   The extraction container according to any one of embodiments 17 to 20, wherein the reservoir is positioned at least either above or below the accommodation area.
[Embodiment 22]
   The extraction container according to any one of embodiments 17 to 20, wherein the reservoir is provided on the outer periphery of the accommodation area.
[Embodiment 23]
   The extraction container according to any one of embodiments 17 to 21, wherein the reservoir communicates with the accommodation area through a thin tube that penetrates at least one of the squeezing surfaces.
[Embodiment 24]
   The extraction container according to any one of embodiments 17 to 23, wherein a groove is provided on the squeezing surface of the squeezing member.
[Embodiment 25]
   The extraction container according to any one of embodiments 17 to 24, having the two squeezing members, wherein the two squeezing members are movable with the squeezing surfaces thereof facing each other, and the space between the squeezing surfaces facing each other of the two squeezing members defines the accommodation area.
[Embodiment 26]
   The extraction container according to embodiment 25, wherein
   one of the two squeezing members has a recess, the inner bottom surface of the recess serves as a squeezing surface,
   the other squeezing member of the two squeezing members is liquid-tightly slidable in the recess with the squeezing surface thereof facing the inner bottom surface.
[Embodiment 27]
   The extraction container according to any one of embodiments 17 to 20 comprises:
   a syringe-type cylinder member,
   a sieve member having pores and disposed so as to partition the hollow space of the cylinder member into a proximal space and a distal space, and
   a piston member liquid-tightly slidable in the proximal space of the cylinder member, wherein
   the sieving member and the piston member serve as the squeezing members, and the inner surfaces thereof facing each other serve as squeezing surfaces,
   at least a part of the proximal space between the squeezing surface of the piston member and the squeezing surface of the sieve member defines the accommodation area, and
   the distal space defines the reservoir.
[Embodiment 28]
   The extraction container according to any one of embodiments 17 to 27, wherein the extract liquid recovered in the reservoir is made to flow or circulate.
[Embodiment 29]
   The extraction container according to any one of embodiments 17 to 28 for use in the method according to any one of embodiments 1 to 16.
[Embodiment 30]
   An extract liquid containing particulate matter in the smoke of a heated smoking article, obtained by the method according to any one of embodiments 1 to 16.
[Embodiment 31]
   The extract liquid according to embodiment 30, containing no solvent.
[Embodiment 32]
   The extract liquid according to embodiment 30 or 31 for use in an *in vitro* toxicity test.
[Embodiment 33]
   The extract liquid according to embodiment 32, wherein the *in vitro* toxicity test is a micronucleus test or an Ames test.
[Embodiment 34]
   An *in vitro* toxicity test method, including using an extract liquid containing particulate matter in the smoke of a heated smoking article obtained by the method according to any one of embodiments 1 to 16.

### ADVANTAGEOUS EFFECTS OF INVENTION

The extraction method of the present invention has made it possible to extract particulate matter from the smoke of a heated smoking article with efficiency higher than that of conventional extraction methods. According to the method of the present invention, extraction with no solvent or a small amount of solvent is possible. Therefore, an *in vitro* toxicity test can be performed without any influence of the solvent. Furthermore, the influence of a solvent can also be grasped by arbitrarily adding the solvent, and a comparative test can also be performed on various types of heated smoking articles.

### BRIEF DESCRIPTION OF DRAWINGS

Fig. 1 is a sectional view of an extraction container according to the first embodiment of the present invention.
Fig. 2 is a plan view of a squeezing surface of a second squeezing member included in the extraction container according to the first embodiment. Fig. 2(a) illustrates an example in which the squeezing surface is a flat surface and Fig. 2(b) illustrates an example in which radial grooves are formed on the squeezing surface.
Fig. 3 is a cross-sectional view of the extraction container according to the first embodiment, the extraction container including a driving means to drive the squeezing member included therein.
Fig. 4 is a view of the extraction container according to the second embodiment of the present invention. Fig. 4(a) is a cross-sectional view of the extraction container and Fig. 4(b) is a schematic diagram of the extraction container including a circulation device for circulating an extract liquid.
Fig. 5 is a cross-sectional view of an extraction container of the first configuration example according to a third embodiment of the present invention.
Fig. 6 is a cross-sectional view of an extraction container of the second configuration example according to the third embodiment of the present invention.
Fig. 7 is a cross-sectional view of an extraction container of the first configuration example according to the fourth embodiment of the present invention.
Fig. 8 is a cross-sectional view of an extraction container of the second configuration example according to the fourth embodiment of the present invention.
Fig. 9 is cross-sectional view of an extraction container according to the fifth embodiment of the present invention.
Fig. 10 is a flowchart of a method for extracting a liquid from filters using the extraction container according to an embodiment of the present invention.
Fig. 11 shows the relationship between compression pressure and release pressure (MPa) (4 times) applied to the filters, and the filter thickness (mm) and the amount of the extract liquid. The filter thickness is denoted by a white circle, and the amount of extract liquid is denoted by a black triangle.
Fig. 12 shows the results of the Ames test. The horizontal axis plots the doses of samples added (µg total particulate matter (TPM) eq./mL), and the vertical axis plots the ratio (%) of the number of viable cells (cells that have undergone cell reverse mutations) after the addition of a sample to the number of viable cells before the addition of the sample.
Fig. 13 shows the result of the MN test. The horizontal axis plots the doses of samples added (µg total particulate matter (TPM) eq./mL), and the vertical axis plots the frequency (%) of the occurrence of micronuclei (MN) (MN induction rate).

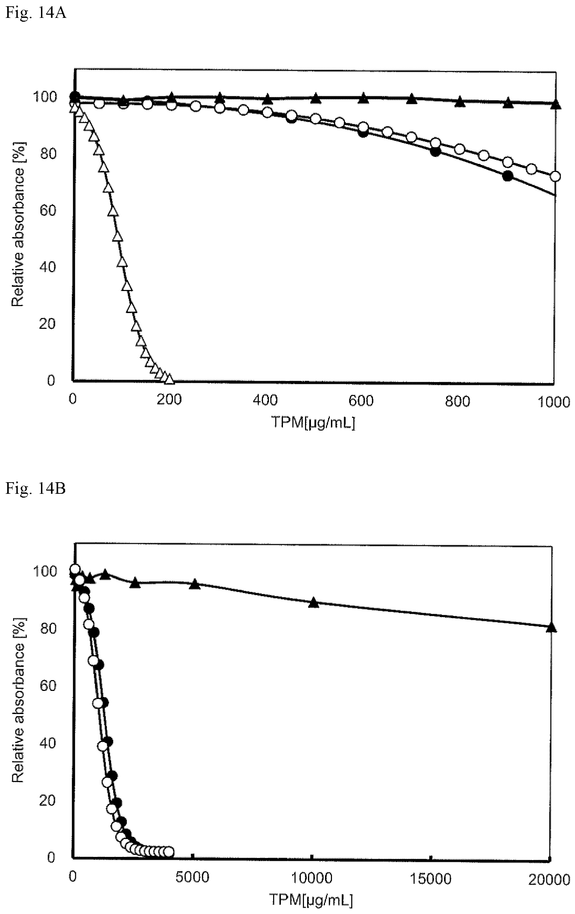

Fig. 14A shows the results of the neutral red uptake activity test using DMSO extraction samples (conventional method). The horizontal axis plots the doses of samples added, and the vertical axis plots the percentage of the neutral red uptake of cells exposed to a sample relative to the neutral red uptake of cells to which no sample was added. Test samples used herein are: cigarette 3R4F (open triangle); heated smoking article Marlboro for iQOS REGULAR (trademark) (sample A) (black circle); heated smoking article B (sample B) (open circle); and Mevius (registered trademark) Regular for Ploom TECH (black triangle).
Fig. 14B shows the results of the neutral red uptake activity test using SEP (Solvent-Free Extraction Process). The horizontal axis plots the doses of samples added, and the vertical axis plots the percentage of the neutral red uptake of cells exposed to a sample relative to the neutral red uptake of cells to which no sample was added. Test samples used herein are: heated smoking article, Marlboro for iQOS REGULAR (trademark) (sample A) (black circle); heated smoking article B (sample B) (open circle); and Mevius (registered trademark) Regular for Ploom TECH (black triangle).
Fig. 14C shows the results of the neutral red uptake activity test using SEP. The horizontal axis plots the doses of samples added, and the vertical axis plots the percentage of the neutral red uptake of cells exposed to a sample relative to the neutral red uptake of cells to which no sample was added. Test samples used herein are: heated smoking article, Marlboro for iQOS REGULAR (trademark) (sample A) (black circle); heated smoking article B (sample B) (open circle); and Mevius (registered trademark) Regular for Ploom TECH (black triangle).
Fig. 14D shows the results of the neutral red uptake activity test using SEP. The horizontal axis plots the doses of samples added, and the vertical axis plots the percentage of the neutral red uptake of cells exposed to a sample relative to the neutral red uptake of cells to which no sample was added. Test samples used herein are: heated smoking article, Marlboro for iQOS REGULAR (trademark) (sample A) (black circle); heated smoking article B (sample B) (open circle); and Mevius (registered trademark) Regular for Ploom TECH (black triangle).

### DESCRIPTION OF EMBODIMENTS

### 1. Method for extracting particulate matter from smoke of heated smoking article

The first aspect of the present invention relates to a method for extracting particulate matter from the smoke of a heated smoking article.
In one embodiment, the method for extracting particulate matter from the smoke of a heated smoking article comprises:
(1) collecting particulate matter in the smoke of a heated smoking article in two or more filters capable of capturing the particulate matter,
(2) stacking the two or more filters, and
(3) squeezing the stacked filters to extract components adhered to the filters.

In contrast to combustible smoking articles such as cigarettes, many "heated smoking articles" have been developed, in which a nicotine-containing aerosol-forming substrate, such as tobacco, is of a heated-type instead of a combustion type. In heated smoking articles, aerosols are produced by heating an aerosol-forming substrate. Known heated smoking articles include, for example, smoking articles in which aerosols are electrical heated or produced by transfer of heat from a combustible fuel element or a heat source to an aerosol-forming substrate. During smoking, volatile compounds are released from the aerosol-forming substrate as a result of heat transfer from a heat source and are then mixed in the air sucked through the smoking article. As the released compounds cool, the compounds condense to form aerosols, which are inhaled by a consumer.

Further, another type of the heated smoking article is also known. In a heated smoking article of this type: vapor is generated by heating directly or indirectly a porous or fibrous material impregnated with alcohol, ethanol, glycerin, propylene glycol or a mixed solution thereof with the use of the above electric heat source or a heat source based on a chemical reaction without burning tobacco; the vapor and particulate matter are mixed to form a gas; and then the gas is mixed in air sucked through the smoking article; further, the gaseous mixture of the vapor, the particulate matter and air passes through the above-mentioned tobacco, or tobacco leaf, reconstituted tobacco, reconstituted tobacco sheet, or reconstituted tobacco granules so that a gas containing smoking flavor components that have contained in tobacco, tobacco leaf, or reconstituted tobaccos, and volatile compounds such as nicotine and various natural flavors is mixed in. In the heated smoking article of this type, a consumer inhales the gaseous mixture containing the volatile compound-containing gas, that is, the gaseous mixture of aerosols (particle phase components) and the gas phase components. Typically, a consumer sucks at an end (the end on the mouth side, or the filter end or the mouthpiece end) of the heated smoking article to inhale the gaseous mixture.

Herein, non-combustion type smoking articles in which nicotine-containing aerosols are produced from a tobacco material, a tobacco extract, or another nicotine source without heating in some cases, for example by a chemical reaction are also referred to as "heated smoking articles".

Herein, the term "heated smoking article" includes all types of heated smoking articles as described above, unless otherwise specified.

The term "particulate matter in the smoke of a heated smoking article" refers to all substances except gaseous and volatile substances in smoke generated from heated smoking articles, and may also be referred to as total particule matter (TPM). In one embodiment, examples of particulate matter in the smoke of a heated smoking article include water vapor, nicotine, propylene glycol, glycerin, and menthol. Particulate matter in smoke generated when one of heated smoking articles, a heated smoking article manufactured by Philip Morris Products Societe Anonym (trade name: Marlboro (registered trademark) HeatSticks (registered trademark): SMOOTH & PURPLE, PURPLE MENTHOL, REGULAR, BALANCED REGULAR, MENTHOL, and MINT (trademark)) is applied to a dedicated heating-type smoking device (trade name: iQOS (registered trademark), iQOS2.4, iQOS2.4plus) contains nicotine, propylene glycol, glycerin, fructose, glucose, and a trace amount of carbon monoxide etc., below the limit of quantification. Components can be analyzed by gas chromatography-mass spectrometry (GC/MS) or the like depending on component types.

The material and shape of the "filter(s) capable of capturing particulate matter" are not particularly limited as long as the filter can capture the particulate matter in the smoke of a heated smoking article. Examples of usable filters include glass fiber filters, HEPA filters, ULPA filters, and Cambridge filters. A glass fiber filter is preferable. The shape is preferably a flat circular shape having a diameter of about 10 mm to 100 mm. More preferably, it is a flat circular shape having a diameter of 40 mm to 50 mm. More preferably, it is a flat circular shape having a diameter of 44 mm. Shapes other than circles, such as squares, diamonds, and triangles can also be used. The thickness of each filter is preferably 4 mm or less, more preferably 2 mm or less.

In one embodiment, the "filter capable of capturing particulate matter" includes a HEPA filter (High Efficiency Particulate Air Filter), which is a type of air filter capable of removing dust, dirt, and the like from air. According to JIS Z 8122, the HEPA filter is specified as "an air filter having a particle collection efficiency of 99% or more for collecting particles having a particle size of 0.3 µm at the rated air volume and an initial pressure loss of 245 Pa or less." In one embodiment, the "filter capable of capturing particulate matter" is a filter that "has a particle collection efficiency of 99% or more for collecting particles having a particle size of 0.3 µm at the rated air volume". A filter having particle collection efficiency higher than that of the HEPA filter is an ULPA filter (Ultra Low Penetration Air Filter). The ULPA filter is specified according to JIS Z 8122 as "an air filter having a particle collection efficiency of 99.9995% or more for collecting particles having a particle size of 0.15 µm at the rated air volume and an initial pressure loss of 245 Pa or less. Filters having the same properties as those of the HEPA filter or the ULPA filter can also be used.

In one embodiment, a Cambridge filter can also be used as the "filter capable of capturing particulate matter". The Cambridge filter is a flat circular glass fiber filter having a diameter of about 44 or 92 mm and a thickness of 1.5 mm, and is well known to and widely used by those skilled in the art as a filter capable of capturing particulate matter. The Cambridge filter is available from, for example, Cambridge Filter Japan, Ltd., Borgwaldt (catalog number 8020 285 2).

"Collecting particulate matter in two or more filters capable of capturing particulate matter" may be performed, for example, by generating smoke from a heated smoking article and directly blowing the generated smoke to the filters. The smoke can be generated from a heated smoking article according to, for example, the CORESTA recommended method No. 81 (NPL 4), a predetermined mechanical smoking method (for example, negative pressure of the mechanical smoking device, puff duration, puff volume, puff frequency of 30 seconds ± 0.5 seconds).

Generation of smoke from a heated smoking article can be performed using, for example, a sample provided according to the humidity control/conditioning method for heated smoking articles defined by ISO (the International Organization for Standardization) 3402: 1999 (NPL 5) according to a predetermined inhalation method of a smoking machine (for example, puff amount: 55 mL/time, puff duration: 2.0 seconds/time, puff frequency: every 30 seconds) and conditions of the start and the end of inhalation as defined by ISO 3308: 2012 (NPL 6).

In view of capturing a larger amount of particulate matter in filters, it is preferable, without limitation, to blow smoke to one filter by one filter; specifically, the following manner is preferable: the first filter is replaced by the next filter when the amount of the particulate matter captured on the first filter reaches a certain level, smoke is blown to the next filter in the same manner, and this cycle is repeated. In one embodiment, replacement is performed when the particulate matter captured by the first filter reaches 300 mg/pad or more, 400 mg/pad or more, 450 mg/pad or more, or 500 mg/pad or more. In one embodiment, replacement is performed when the particulate matter captured by the first filter is 800 mg/pad or less, 750 mg/pad or less, 700 mg/pad or less, or 650 mg/pad or less. In one embodiment, the first filter is replaced when the particulate matter captured by the first filter reaches the range of 500-650 mg/pad.

In one embodiment, 3 or more, 5 or more, 7 or more, 9 or more, or 10 or more filters are used, without limitation. In one embodiment, 25 or less, 23 or less, 20 or less, 18 or less, or 15 or less filters are used. In one embodiment, 10 to 15 filters are used.

The method for extracting particulate matter from the smoke of a heated smoking article includes "(3) squeezing the stacked filters to extract components adhered to the filters."

Without being bound by theory, the method involves squeezing the filters to press and loosen the filters, allowing the particulate matter (components adhered to the filters) captured by the filters to detach from the filters together with the exuded liquid (squeezed-out liquid), and then dissolving the detached particulate matter in the exuded liquid (squeezed-out liquid). Means to "squeeze" is not particularly limited as long as it can press and loosen the filters to exude the liquid.

In one embodiment, step (3) is performed by
(3-i) compressing and releasing the stacked filter,
(3-ii) compressing the stacked filters to extract components adhered to the filters, pouring the extract liquid back to the stacked filters, and then compressing them again, or
(3-iii) performing (3-i) and (3-ii) in combination.

In one embodiment, the compression and release of the stacked filters in step (3-i) may be repeated two or more times. The compression and release of the stacked filters is preferably repeated two or more times, three or more times, or four or more times, without limitation.

In one embodiment, compression is preferably performed at 0.01 MPa or more, 0.1 MPa or more, 0.5 MPa or more, or 2.0 MPa or more. In one embodiment, compression is preferably performed at 0.5 MPa or less, 2.0 MPa or less, 5.0 MPa or less, or 10.0 MPa or less. It is desirable to employ the pressure such that the fluidity of the squeezed-out liquid is not lost. For example, squeezing may be started at 0 MPa and the squeezing pressure may be gradually increased. Squeezing is performed in the range of 0 MPa-10.0 MPa, for example. When the compression-and-release in step (3-i) is performed two or more times, pressures employed may be the same or different. The pressure of squeezing may be increased in each compression-andrestoration.

In one embodiment, step (3-ii) of compressing the stacked filters to extract components adhered to the filters, pouring the extract liquid back onto the stacked filters, and then compressing them again may be repeated two or more times. Preferably Step (3-ii) is preferably repeated two or more times, three or more times, or four or more times, without limitation.

(3-i) and (3-ii) may be performed singly or in combination. By combining (3-ii) with (3-i), the pressure and/or frequency of compression (pressurization) in (3-i) can be reduced.

In one embodiment, the filters and the extract liquid may be shaken together during steps (3-i) and/or (3-ii). The degree (strength) of shaking may be, without limitation, an amplitude of 2 cm to 6 cm, and 100 to 300 reciprocations per minute. In one embodiment, the degree of shaking may be 4 cm and 200 reciprocations per minute. By shaking the filters and the extract liquid together, an oscillating flow is generated in the extract liquid, and the flow repeatedly collides with the squeezed filters, so that the extraction effect can be further improved.

In one embodiment, step (3) is performed on the filters accommodated in an extraction container having two squeezing surfaces.

In one embodiment, in step (3), the extract liquid obtained by squeezing is made to flow or circulate.

In one embodiment, it is preferable to repeat step (3) until the thickness of the stacked filters becomes equal to or less than the predetermined thickness. Preferably, step (3) is repeated until the thickness becomes 1/2 or less, 1/3 or less, or 1/4 or less than the thickness before performing step (3). Preferably, step (3) is repeated until the filter is twisted or broken down by squeezing.

In one embodiment of the method for extracting particulate matter from the smoke of a heated smoking article, no solvent is used in step (3). In one embodiment, a small amount of an amphiphilic solvent may be added in step (3). The amount of an amphipathic solvent to be added is not particularly limited. In one embodiment, it is 400% (weight/volume) or less, more preferably 200% (weight/volume) or less, based on the filter volume. In one embodiment, the amount of an amphiphilic solvent to be added is greater than 0% (weight/volume), or greater than or equal to 10% (weight/volume).

Examples of amphiphilic solvents include, but are not limited to, dimethyl sulfoxide (DMSO), ethanol, methanol, isopropanol, and acetone.

When no solvent or only a small amount of an amphipathic solvent is used in step (3), various effects on the living body can be examined without the influence of the solvent in, for example, an *in vitro* toxicity test method, which will be described later. Alternatively, if a solvent is arbitrarily added, the influence of the solvent can be grasped, and a comparative test can also be performed on various types of heated smoking articles. In a liquid-type electronic cigarette, results of, for example, comparison between an undiluted solution (liquid) and aerosols collected after smoking can be obtained.

Step (3) may be further followed by
(4) centrifuging the filters to obtain a separated liquid; and
(5) mixing the separated liquid with the extract liquid obtained in step (3).

Centrifugation is not particularly limited in terms of intensity (rotation speed, time) as long as it can separate liquid components remaining in the filters even after squeezing. The rotation speed ranges from, for example, 1,000 rpm to 6,000 rpm, and preferably 3,500 rpm to 5,000 rpm. The time is, for example, 1 minute or longer, and preferably 5 minutes or longer. In one embodiment, for example, centrifugation may be performed at 4000 rpm for 5 minutes.

The separated liquid obtained by centrifugation was mixed with the squeezed-out liquid obtained in step (3) to obtain an extract liquid.

Herein, the liquid obtained in step (3) is referred to as a "squeezed-out liquid" or an "extract liquid". Depending on the context, a mixture of the "squeezed-out liquid" and the "separated liquid" obtained by centrifugation may be referred to as an "extract liquid".

### 2. Extraction container

The second aspect of the present invention relates to an extraction container. The extraction container is useful for extracting particulate matter from the smoke of heated smoking articles. In one embodiment, the extraction container can be used in a method for extracting particulate matter from the smoke of a heated smoking article as described herein. Hereinafter, each of the first to fifth embodiments of the "extraction container", which is the second aspect of the present invention will be described.

### (First embodiment)

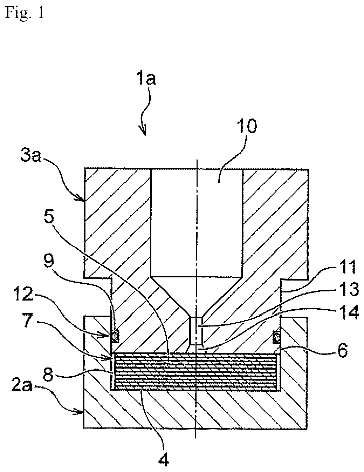

Fig. 1 shows an extraction container 1a according to the first embodiment. The extraction container 1a includes a first squeezing member 2a and a second squeezing member 3a.

The first squeezing member 2a includes a substantially cylindrical recess 6, and the inner bottom surface of the recess 6 serves as the first squeezing surface 4. The second squeezing member 3a has a tip portion 11 formed so as to be inserted into the recess 6 of the first squeezing member 2a. The tip portion 11 has a groove 9 formed along the outer periphery of the side surface thereof, and an O-ring 12 is fitted in the groove 9 for engagement with the inner surface of the recess 6. Specifically, the second squeezing member 3a is formed so as to be liquid-tightly slidable in the recess 6 of the first squeezing member 2a.

The first squeezing surface 4 and the second squeezing surface 5 of the second squeezing member disposed to face the said first squeezing surface 4 define the accommodation area 8 between them. The accommodation area 8 can accommodate two or more filter members 7 in a stacked state. When the "extraction container" is used in the "method for extracting particulate matter from the smoke of a heated smoking article", which is the first aspect of the present invention described above, the filters described for the first and third aspects of the present invention and those described in examples can be used as the filter members 7.

A reservoir 10 is formed on the second squeezing member 3a, and the reservoir 10 communicates with the accommodation area 8 through a thin tube 13 extending from the reservoir 10. In the thin tube 13, a trumpet-shaped opening 14 may be formed in the vicinity of the second squeezing surface 4 in order to facilitate the recovery of the liquid in the accommodation area 8.

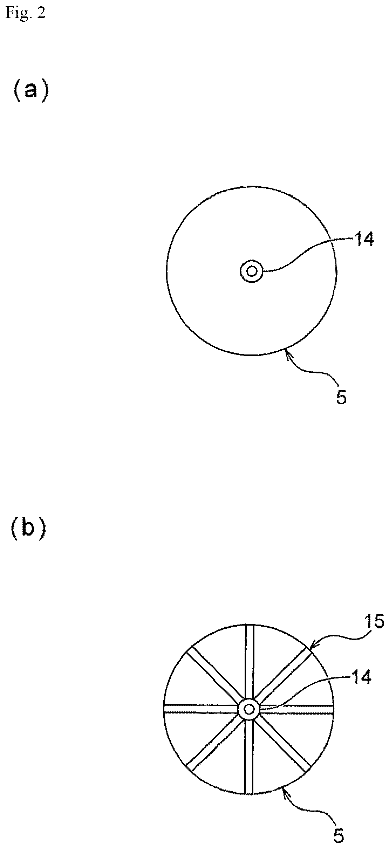

The second squeezing surface 5 of the second squeezing member 3a may be formed in a flat shape as shown in Fig. 2 (a) in a region other than the opening 14. More preferably, as shown in Fig. 2(b), a plurality of grooves 15 are formed radially in the radial direction around the opening 14 as the center.

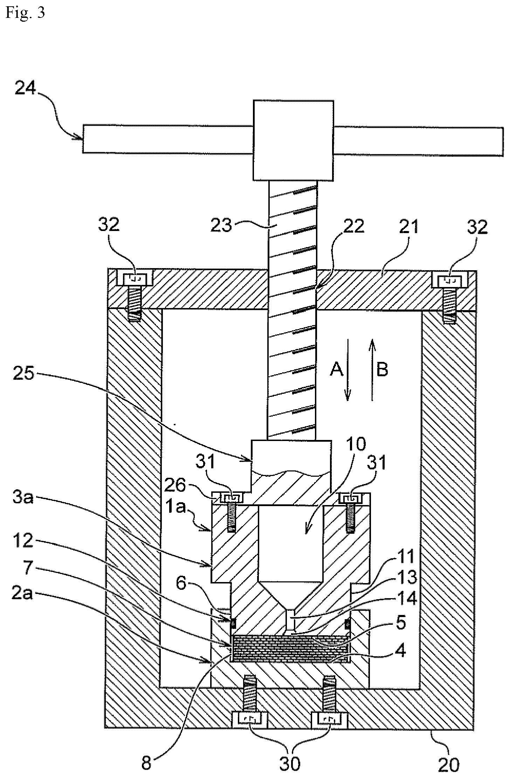

As described above, the second squeezing member 3a is liquid-tightly slidable in the recess 6 of the first squeezing member 2a. Accordingly, the first squeezing member 2a and the second squeezing member 3a can be moved relative to each other and specifically, the first and second squeezing surfaces 4, 5 can be moved closer to each other or separated from each other. The driving means to cause this relative movement of the first and second squeezing members 2a, 3a can be, for example, the configuration shown in Fig. 3.

As shown in Fig. 3, the extraction container 1a with the driving means includes a housing 20 for housing the extraction container 1a, a lid portion 21 of the housing 20, a screw through hole 22 formed at the center of the lid portion 21, a screw rod 23 to be screwed into the screw through hole 22 having a screw thread formed on the inner surface thereof, a handle 24 attached to an end portion on the side of the screw rod 23 projecting from the housing 20, and a bearing portion 25 rotatably supporting about an axis the end portion within the housing 20 of the screw rod 23 and fixed to the opening top of the reservoir 10 of the second squeezing member 3a.

The first squeezing member 2a of the extraction container 1a is fixed to the bottom plate of the housing 20 with a bolt 30, the second squeezing member 3a is fixed to the overhanging bottom plate 26 of the bearing portion 25 with a bolt 31, and the lid portion 21 is fixed to the housing 20 with bolts 32.

When the handle 24 is rotated in the direction in which the screw rod 24 is pulled into the housing 20 (direction A in Fig. 3), the screw rod 24 moving in direction A moves the second squeezing member 3a downward via the bearing portion 25. At this time, since the second squeezing surface 5 approaches the first squeezing surface 4, the plurality of stacked filter members 7 placed between the first and second squeezing surfaces 4 and 5 are compressed. In the driving means of Fig. 3, the pressure associated with screwing the screw rod 23 into the screw hole 22 using the handle 24 having a large diameter can be used as the compression force for compressing the filter members 7, so that a large compression force can be obtained.

On the other hand, when the handle 24 is rotated in the direction in which the screw rod 24 is pulled out from the housing 20 (direction B in Fig. 3), the screw rod 24 moving in direction B moves the second squeezing member 3a upward via the bearing portion 25. At this time, since the second squeezing surface 5 is moved away from the first squeezing surface 4, the plurality of stacked filter members 7 placed between the first and second squeezing surfaces 4 and 5 are released from the above-described compression.

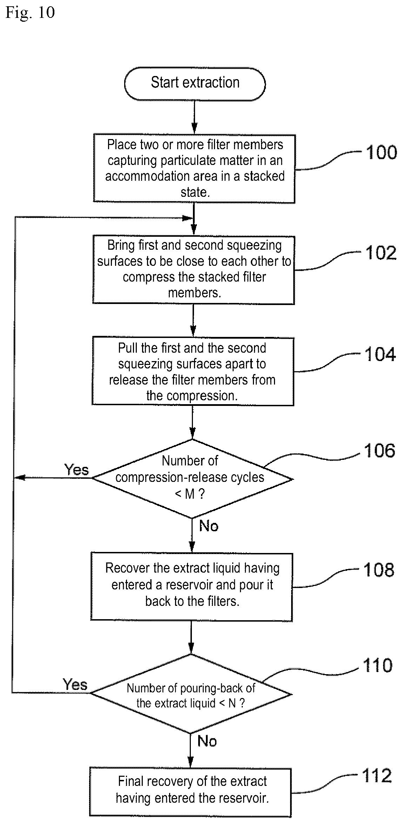

Next, a method for extracting a liquid from the filter members 7 using the extraction container with the driving means shown in Fig. 3 will be described with reference to the flowchart in Fig. 10.

As shown in Fig. 10, first, two or more filter members 7 that have captured the particulate matter are stacked and placed in the accommodation area 8 (step 100).

Next, the first squeezing surface 4 and the second squeezing surface 5 are brought to be close to each other to compress the stacked filter members 7 (step 102). When the driving means shown in Fig. 3 is used, step 102 is performed by rotating the handle 24 in the direction in which the screw rod 24 is pulled into the housing 20 (direction A in Fig. 3), as described above.

Next, the first squeezing surface 4 and the second squeezing surface 5 are pulled apart to release the stacked filter members 7 from the compression (step 104). When the driving means shown in Fig. 3 is used, step 104 is performed by rotating the handle 24 in the direction in which the screw rod 24 is pulled out from the housing 20 (direction B in Fig. 3), as described above. The compression process of step 102 and the release process of step 104 are count as one compression-release cycle. As preferable pressure at the time of compression and release, the pressure described above for the first aspect of the present invention or the pressure described later in the third aspect of the present invention is used.

Next, it is determined whether or not the number of compression-release cycles performed in steps 102 and 104 is less than M (step 106). Here, M is set to an integer of 2 or more.

When the number of compression-release cycles is less than M (affirmative determination in step 106), the step loops back to step 102 and the compression-release cycle is repeated again. By repeating the compression-release cycle, the filter members 7 are squeezed and a liquid containing the captured particulate matter is extracted. The extract liquid flows from the opening 14 through the thin tube 13 into the reservoir 10. At this time, when the second squeezing surface 5 includes the grooves 15 as shown in Fig. 2(b), the extract liquid more smoothly passes through the grooves 15, passes from the opening 14 through the thin tube 13, and thus enters the reservoir 10.

When the number of compression-release cycles reaches M (negative determination in step 106), the step proceeds to the next step 108. In step 108, the extract liquid having entered the reservoir 10 is recovered and poured back onto the filter members 7 in the accommodation area 8. This pouring-back process is counted as a single process. The recovery and pouring-back of the extract liquid having entered the reservoir 10 are performed using, for example, a pipette. Further, it is preferable to restore the state of the first squeezing surface 4 and the second squeezing surface 5 from the separation to the close proximity to thereby allow a larger amount of the extract liquid to exude and enter the reservoir 10, before collecting the extract liquid.

Next, it is determined whether or not the number of pouring-back performed in step 108 is less than N (step 110). Here, N is set to an integer of 2 or more.

When the number of pouring-back of the extract liquid is less than N (affirmative determination in step 110), the step loops back to step 102 again, the compression-release cycle is repeated M times, and the extract liquid is recovered and poured back.

When the number of pouring-back of the extract liquid reaches N (negative determination in step 110), the extract liquid having entered the reservoir 10 is finally recovered with a pipette or the like. The recovered extract liquid is used for the analysis of particulate matter.

The following changes can also be made in the flowchart of Fig. 10 (the same applies to other embodiments described below).

A step of shaking the extraction container 1a can be added after the compression-release cycle in steps 102 and 104 in Fig. 10. The direction in which the extraction container 1a is shaken is preferably, for example, the lateral direction along the squeezing surface (in the example in Fig. 3, the direction perpendicular to the axis direction of the screw rod 23), but is not limited thereto. By shaking the extraction container 1a, an oscillating flow is generated in the extract liquid, and the flow repeatedly collides with the squeezed filter members 7, so that the extraction effect can be further improved.

Further, in step 102 in Fig. 10, a process of rotating the squeezing surfaces 4 and 5 relative to each other may be added to the compression by the approach of the squeezing surfaces 4 and 5. By rotating the squeezing surfaces 4 and 5 relative to each other, not only the compression force but also the twisting force is applied to the stacked filter members 7, and thus the liquid extraction effect can be further improved.

Furthermore, in step 102 in Fig. 10, an amphipathic solvent may be added to the filter members 7 for performing compression, as described above in the "method for extracting particulate matter from the smoke of heated smoking articles". Of course, compression without the use of a solvent is also possible.

Further, in the example in Fig. 10, the pouring-back process is performed N times for each of the compression-release cycles, the number of which is M; that is, a total of M × N compression-release cycles are performed. The pouring-back process may also be performed in the compression-release cycles. In this case, step 108 is placed between steps 104 and 106, and step 110 is deleted, in the flowchart in Fig. 10. The timing of the pouring-back process can be changed in an appropriate way other than the above, and the embodiment of the present invention includes an arbitrary combination of the compression-release cycle and the pouring-back process.

Further, in the driving means shown in Fig. 3, squeezing is performed by rotating the handle 24 manually, but an electric driving means such as a motor may be used instead of the handle 24. Further, the filter members 7 may be squeezed by squeezing members directly or indirectly using a piezoelectric actuator.

Furthermore, in the above example, the second squeezing member 3a was moved; however, the first squeezing member 2a may be moved, or both of the first and the second squeezing members may be moved.

Furthermore, in step 106, conditions can also be changed so that the compression-release cycles are continued until the thickness of the stacked filter members 7 becomes a thickness in a predetermined ratio (for example, 1/3) or less to that before the start of the compression-release cycle.

Regarding the extraction container 1a, the reservoir 10 is formed inside the second squeezing member 3a; however, the reservoir 10 may be formed inside the first squeezing member 2a, or may be formed in both of the first squeezing member 2a and the second squeezing member 3a.

### (Second embodiment)

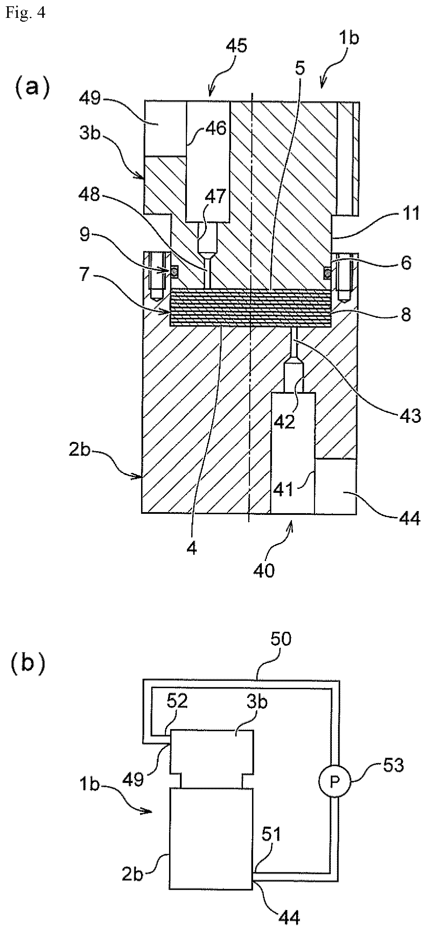

In the first embodiment, after the extract liquid is stored in the reservoir 10, the stored extract liquid is poured back again on the filter members 7 using a pipette or the like. The second embodiment relates to an extraction container including a mechanism for circulating an extract liquid without using a pipette, which will be described below with reference to Fig. 4. Regarding the same constituents as those in the first embodiment, the same symbols are applied thereto and detailed descriptions thereof are omitted, while only differences are described.

As shown in Fig. 4(a), the extraction container 1b according to the second embodiment includes the first squeezing member 2b and the second squeezing member 3b. The second squeezing member 3b is formed so as to be liquid-tightly slidable in the recess 6 of the first squeezing member 2b, and the first squeezing surface 4 and the second squeezing surface 5 define the accommodation area 8 between them. The accommodation area 8 is the same as that in the first embodiment in that it can accommodate two or more filter members 7 in a stacked state.

The first squeezing member 2b has the first cavity portion 40 that functions as a tube connector for a circulation tube described later, and the second squeezing member 3b has the second cavity portion 45 that functions as a tube connector for the circulation tube.

In the first cavity portion 40, a large-diameter portion 41, a medium-diameter portion 42, and a small-diameter portion 43, having three different diameters, respectively, are formed. The large-diameter portion 41 opens at the bottom of the first squeezing member 2b, and is connected to an escape port 44 of the circulation tube, which is formed as a cutout on the side surface of the first squeezing member 2b. The small-diameter portion 43 opens at the first squeezing surface 4, whereby the first cavity portion 40 communicates with the accommodation area 8.

Similarly, in the second cavity portion 45, a large-diameter portion 46, a medium-diameter portion 47, and a small-diameter portion 48, having three different diameters, respectively, are formed. The large-diameter portion 46 opens at the top of the second squeezing member 3b and is connected to an escape port 49 of the circulation tube, which is formed as a cutout on the side surface of the second squeezing member 3b. The small-diameter portion 48 opens at the second squeezing surface 5, whereby the second cavity portion 45 communicates with the accommodation area 8.

Fig. 4(b) shows a state in which a circulation tube 50 is fitted to the extraction container 1b. As shown in Fig. 4(b), one end portion 51 of the circulation tube 50 is inserted into the large-diameter portion 41 of the first cavity portion 40, and the other end portion 52 of the circulation tube 50 is inserted into the large-diameter portion 46 of the second cavity portion 45, so as to fit the circulation tube 50 to the extraction container 1b. A peristaltic pump 53 is attached to the circulation tube 50. When the peristaltic pump 53 is driven to suck from one end portion 51 of the circulation tube 50, the extract liquid in the accommodation area 8 is sucked by the circulation tube 50 through the small-diameter portion 43 and the medium-diameter portion 42 of the first cavity portion 40, passes through the circulation tube 50, is discharged from the other end portion 52, and then flows into the accommodation area 8 again via the relay portion 47 and the small-diameter portion 48 of the second cavity portion 45. Of course, it is also similarly possible to suck the extract liquid from the other end portion 52 of the circulation tube 50. While the peristaltic pump 53 is being driven, the extract liquid flows and circulates in the squeezed filter members 7, so that the liquid extraction effect can be further improved.

The driving means shown in Fig. 3 may also be attached to the extraction container 1b according to the second embodiment, for example. At this time, the direction of the circulation tube can be changed by bending one end portion 51 of the circulation tube 50 at the escape port 44. Therefore, even if the bottom surface of the first squeezing member 2b is attached to, for example, the housing 20 in Fig. 3, it is possible to avoid this and appropriately dispose the circulation tube 50. Similarly, even if the bearing portion 25 in Fig. 3 is attached to the top of the second squeezing member 3b, it is possible to avoid this and appropriately disposed the circulation tube 50 by bending the other end portion 52 of the circulation tube 50 at the escape port 49.

In the extraction container 1b according to the second embodiment, the first cavity portion 40 and the second cavity portion 45, in particular, the medium-diameter portions 42, 47 and the small-diameter portions 43, 48 also play the role as the reservoir 10 in the first embodiment. In the second embodiment, it is possible to more efficiently and effectively perform the "pouring-back of the extract" in the first embodiment by using the circulation hose 50 and the peristaltic pump 53.

A liquid can be extracted from the filter members 7 according to the flowchart in Fig. 10 by using the extraction container 1b according to the second embodiment. In this case, step 108 "recover the extract liquid and pour it back to the filters" in Fig. 10 corresponds to the "circulation of the extract liquid through the circulation hose 50 and the accommodation area 8" by the peristaltic pump 53. In addition, the "number of pouring-back of the extract" in step 110 may be, for example, the number of times that the process of "driving the peristaltic pump 53 for a predetermined time to circulate the extract", which is counted as a single process, is performed or the number of times that the process of "circulating once the extract liquid from the accommodation area 8 to the accommodation area 8 via the circulation tube 50", which is counted as a single process, is performed.

In step 112, any of the end portions 51 and 52 of the circulation tube 50 on the discharge side of the extract liquid is removed from the extraction container 1b, and then the extract liquid is discharged from the removed end portion to a container for analysis, for example, using the peristaltic pump 53, so that the extract liquid is finally recovered.

Since the extraction effect is improved in the second embodiment as described above, it is also possible to set the threshold M in step 106 and the threshold N in step 110 at low levels, for example.

### (Third Embodiment)

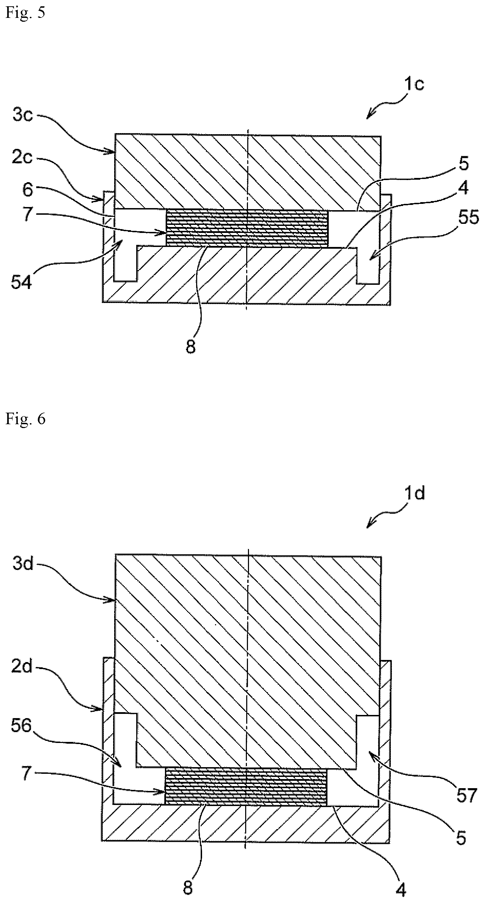

In the first and second embodiments, the reservoir is formed inside the first squeezing member or the second squeezing member. The third embodiment has a configuration in which a reservoir is provided on the outer periphery of the accommodation area 8 of the filter members 7, and will be described below with reference to Figs. 5 and 6. Regarding the same constituents as those in the first embodiment, the same symbols are applied thereto and detailed descriptions thereof are omitted, while only differences are described.

Fig. 5 shows an extraction container 1c according to the first configuration example of the third embodiment. The extraction container 1c includes the first squeezing member 2c and the second squeezing member 3c similarly to the first embodiment. The second squeezing member 3c is formed so as to be liquid-tightly slidable in the recess 6 of the first squeezing member 2c, and the first squeezing surface 4 and the second squeezing surface 5 defines the accommodation area 8 between them. The accommodation area 8 is the same as that in the first embodiment in that it can accommodate two or more filter members 7 in a stacked state.

In the third embodiment, there is an empty space on the outer periphery of the accommodation area 8, and this entire space serves as a reservoir 54. The reservoir 54 has an annular groove 55 formed on the outer periphery of the first squeezing surface 4. When the filter members 7 are pressed to extract a liquid, the volume of the space right next to the filter members 7 of the reservoir 54 may become smaller and unable to store the extract liquid. Even in such a case, the extract liquid can be stored in the annular groove 55.

Fig. 6 shows an extraction container 1d according to the second configuration example of the third embodiment. The extraction container 1d includes the first squeezing member 2d and the second squeezing member 3d, and has a reservoir 56 on the outer periphery of the accommodation area 8. The reservoir 56 of the extraction container 1d has an annular groove 57 on the outer periphery of the second squeezing surface 5 in contrast to the annular groove 55 formed on the first squeezing surface 4 in the first configuration example. Except this, the same in the first configuration example also applies to the second configuration example.

### (Fourth Embodiment)

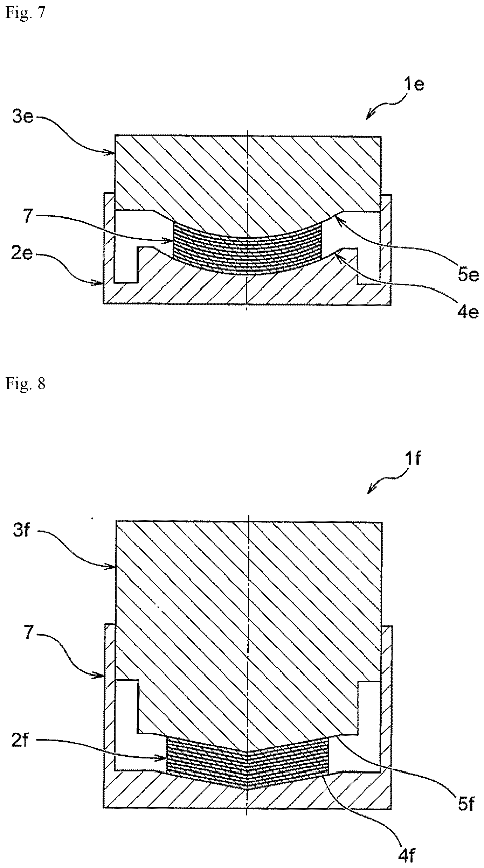

In the first to third embodiments, the squeezing surfaces 4 and 5 are formed as flat surfaces, but the present invention is not limited thereto. Examples in which the squeezing surfaces are not flat surfaces will be described as the fourth embodiment with reference to Figs. 7 and 8.

Fig. 7 shows an extraction container 1e according to the first configuration example of the fourth embodiment. The extraction container 1e has the same basic configuration as the extraction container 1c shown in Fig. 5, except that the first squeezing surface 4e and the second squeezing surface 5e are not flat surfaces but spherical surfaces. In the example in Fig. 7, the spherical surface of the first squeezing surface 4e is formed concave and the spherical surface of the second squeezing surface 5e is formed convex; however, the configuration may be reversed. Preferably, the radius of the spherical surface of the first squeezing surface 4e and that of the second squeezing surface 5e are substantially the same so that both squeezing surfaces can be fitted to each other. The "spherical surface" in the fourth embodiment may be a convex (or concave) curved surface as a whole, and another quadric surface such as an elliptic surface, a paraboloid, or a hyperboloid, or a quadric or higher-order curved surface may be used.

Fig. 8 shows an extraction container 1f according to the second configuration example of the fourth embodiment. The extraction container 1f has the same basic configuration as the extraction container 1d shown in Fig. 6, except that the first squeezing surface 4f and the second squeezing surface 5f are not flat surfaces but conical slopes. In the example in Fig. 8, the conical slope of the first squeezing surface 4f is formed concave and the conical slope of the second squeezing surface 5f is formed convex; however, the configuration may be reversed. Preferably, the slope angles of the first squeezing surface 4f and the second squeezing surface 5f are substantially the same so that both squeezing surfaces can be fitted to each other.

Needless to say, the shapes of the first and second squeezing surfaces according to the fourth embodiment are applicable not only to the third embodiment but also to other embodiments.

### (Fifth Embodiment)

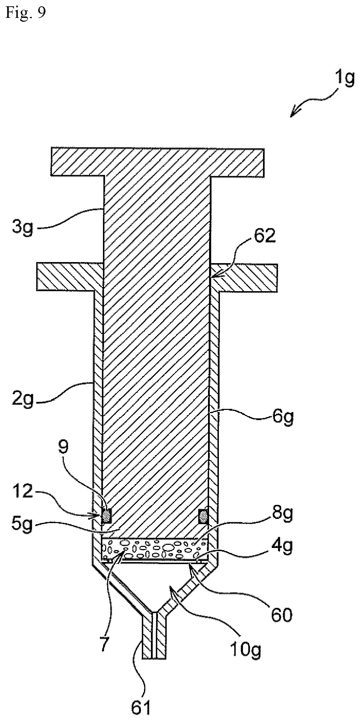

The fifth embodiment relates to a syringe-type extraction container, which will be described below with reference to Fig. 9.

As shown in Fig. 9, an extraction container 1g according to the fifth embodiment includes a syringe-type cylinder member (first squeezing member) 2g having a hollow space 6g (recess) and a piston member (second squeezing member) 3g.

The cylinder member (first squeezing member) 2g includes: a sieve member 60 having pores (thin tubes) and disposed so as to partition the hollow space 6g (recess) into a proximal space 62 and a distal space (reservoir) 10g ; and a tip portion 61 in which a tube for ejecting the extract liquid is formed.

A groove 9 is formed along the outer periphery of the side surface of the piston member (second squeezing member) 3g, and an O-ring 12 is fitted in the groove 9 so as to engage with the inner surface of the proximal space 62. Specifically, the piston member (second squeezing member) 3g is liquid-tightly slidable in the proximal space 62 of the cylinder member (first squeezing member) 2g.

The sieve member 60 and the piston member (second squeezing member) 3g have their inner facing surfaces serving as a first squeezing surface 4g and a second squeezing surface 5g, respectively. At least a part of the proximal space 62 between the first squeezing surface 4g and the second squeezing surface 5g defines the accommodation area 8g of the stacked filter members 7.

Also, in the syringe-type extraction container 1g according to the fifth embodiment, the piston member (second squeezing member) 3g is made to slide in the cylinder member (first squeezing member) 2g, so that the compression-release cycle described in the first embodiment in Fig. 10 can be performed on the filter members 7 in the accommodation area 8g. A liquid is extracted from the filter members 7 subjected to the compression-release cycle, and the extract liquid is exuded from the pores of the sieve member 60 to the distal space (reservoir) 10g. At this time, the extraction container 1g can be disposed with the tip portion 61 facing downward and the opening of the tip portion 61 closed to thereby store the extract liquid in the distal space (reservoir) 10g. In the poring-back process of step 108 in Fig. 10, the extraction container 1g can be disposed with the tip portion 61 facing downward conversely to thereby allow the extract liquid stored in the distal space (reservoir) 10g to pass through the pores of the sieve member 60 and thus pour back to the filter members 7 again due to gravity.

Since the extraction container 1g according to the fifth embodiment is configured as a syringe type, the compression-release cycle and the pouring-back process can be performed very conveniently, and the operability can be greatly improved.

The embodiments of the present invention have been described above, but the present invention is not limited to only the above examples, and can be arbitrarily or preferably changed within the scope of the present invention.

### 3. Extract liquid containing particulate matter in smoke of heated smoking article

The third aspect of the present invention relates to an extract liquid containing particulate matter in the smoke of a heated smoking article, the extract liquid obtained by a method for extracting particulate matter from the smoke of a heated smoking article.

In one embodiment, the extract liquid contains particulate matter in the smoke of a heated smoking article at a sufficient concentration effective for being subjected to an *in vitro* toxicity test described later. The "sufficient concentration effective for subjecting to an *in vitro* toxicity test etc." means, for example, that the cytotoxicity at the highest concentration is 55 ± 5% in terms of a predetermined cytotoxicity parameter.

In one embodiment, particulate matter in the smoke of a heated smoking article contained in an extract liquid includes one or more substances selected from water vapor, nicotine, propylene glycol, glycerin, menthol, fructose, glucose and carbon monoxide. Preferably, the extract liquid contains these particulate matters in a proportion that reflects the proportion thereof contained in the smoke of the heated smoking article.

In one embodiment, the extract liquid contains 40% (weight/weight) or more, preferably 45% (weight/weight) or more of "glycerin + PG + water."

In one embodiment, the extract liquid contains no solvent. In one embodiment, the extract liquid may contain only a small amount of an amphipathic solvent. In one embodiment, the extract liquid may contain 400 wt% or less, preferably 200 wt% or less of an amphiphilic solvent. The extract liquid containing no solvent or containing only a small amount of an amphipathic solvent can avoid the influence of the solvent in the *in vitro* toxicity test method described later, for example. Furthermore, if a solvent is arbitrarily added, the influence of the solvent can be grasped, and a comparative test can be performed on various types of heated smoking articles.

In one embodiment, the extract liquid is for use in an *in vitro* toxicity test. In one embodiment, the *in vitro* toxicity test is a micronucleus test or an Ames test. The *In vitro* toxicity test is described in detail in "4. *In vitro* toxicity test method."

### 4. In Vitro toxicity test method

The present invention also relates to an *in vitro* toxicity test method. The *in vitro* toxicity test method comprises using an extract liquid containing particulate matter in the smoke of a heated smoking article obtained by the method of the present invention.

*In vitro* toxicity test methods include a bacterial reverse mutation test (Ames test), a micronucleus test (MN test), and a neutral red test, etc.

In the bacterial reverse mutation test (Ames test), point mutations are detected using *Salmonella* and *Escherichia coli* strains auxotrophic for amino acids. This point mutation involves substitution, addition or deletion of one, two, or three DNA base pairs. The principle of this test is to detect a mutation in which the mutation that the test strain has possessed is reverted to restore the functional ability to synthesize essential amino acids. Revertant strains are detected by their ability to grow in the absence of amino acids required by the parental test strain. This test is called a cell reverse mutation test because a bacterium modified to be unable to synthesize amino acids will restore its original ability to synthesize amino acids due to mutation. It may also be referred to as the Ames test, and this name comes from the developer of this test, Professor Ames of the University of California, United States. The Ames test can be performed, for example, according to "Bacterial reverse mutation test" (OECD/OCDE TG471) of "OECD Guidelines for the Testing of Chemicals" (adopted July 21, 1997).

The micronucleus test (MN test) is a genotoxicity test for detecting micronuclei (MN) in the intracellular chambers of interphase cells. Micronuclei arise from acentric chromosomal fragments (lack of centromeres) or whole chromosomes that cannot move to the poles of the cells during late cell division. In this test, cells undergoing cell division is exposed to a test substance, and then the chromosomal aberration-inducing activity or aneuploidy-inducing activity of the chemical substance during or after the exposure is detected. The MN test can be performed according to, for example, "IN VITRO mammalian cell micronucleus test" (OECD/OCDE TG487) of "OECD Guidelines for the Testing of Chemicals" (adopted September 26, 2014).

### EXAMPLES

Hereinafter, the present invention will be described in detail by way of examples, but the present invention is not limited to these examples. Those skilled in the art can easily modify and change the present invention based on the description herein, and those are included in the technical scope of the present invention.

### Example 1 Extraction of particulate matter from smoke of heated smoking article by solventless extraction process

In this example, water recovery rates and the composition of main components as a result of extraction of particulate matter in the smoke of heated smoking articles by a solventless extraction process (SEP) were examined.

Using a smoking device (manufactured byBorgwalt, product name: Smoking Machine, product number: RM20H), smoke was generated from heated smoking articles (a type of producing aerosols by the transfer of heat from a heat source to an aerosol-forming substrate, heated smoking articles manufactured by Philip Morris Products Societe Anonyme (trade name: Marlboro (registered trademark) HeatSticks (registered trademark) SMOOTH REGULAR) by a predetermined inhalation method (puff volume: 55 mL/time, puff duration: 2 seconds/time, puff frequency: 30 seconds) using a dedicated heating-type smoking device (trade name: iQOS (registered trademark), iQOS2.4, Phillip Morris Co., Ltd.). Smoke components were collected by blowing to a Cambridge filter (CF) (obtained from Borgwalt). Specifically, the first CF was replaced to the next CF when the first CF reached 500-650 mg/pad. Through repetition of this procedure, smoke components were collected continuously in 10 to 15 CFs.

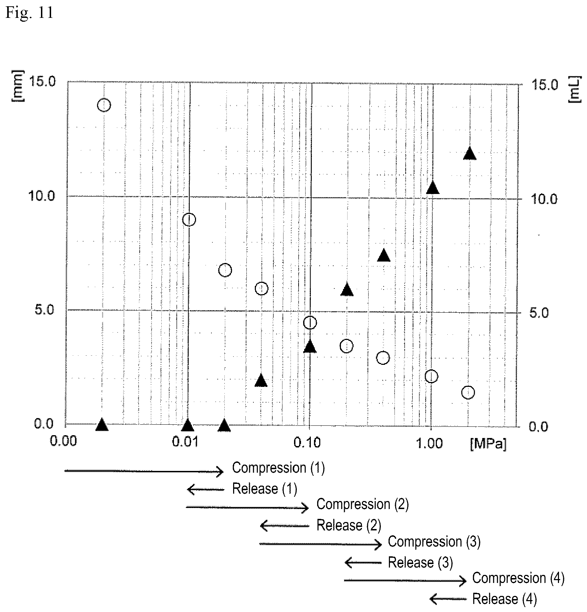

The obtained 10 to 15 CFs were stacked and accommodated in a sealed pressure vessel (Figs. 5, 6, 7, and 8) having an inner size larger than the maximum size of the CF by 20 mm. The accommodated CFs were squeezed by repeating compression (press, rightward arrow) and release (release, leftward arrow) 4 times according to the schedule shown in Fig. 11. The CFs and the squeezed-out liquid coming out of the CFs by squeezing were particularly shaken in the sealed pressure vessel. Shaking was performed at 4 cm and 200 reciprocations per minute. After squeezing, CFs were taken out of the solution and centrifuged (4000 rpm, 5 minutes) to obtain a separated liquid. The squeezed-out liquid and the separated liquid were mixed to obtain an extract.

Fig. 11 shows changes in the CF thickness and the amount of extract liquid (mL) at each time of the first to fourth compression-release cycles. The results in Fig. 11 are each average value of the results of triplicate experiments. In these experiments, the fluidity of the squeezed-out liquid disappears during shaking if excessive compression is applied, and accordingly 10 Mpa was the upper limit.

In this example, an extract liquid containing particulate matter at a concentration of 1000 mg/mL was prepared.

### Example 2 Water recovery rate in extraction of particulate matter from smoke of heated smoking article by solventless extraction process

In the extraction method of Example 1, 8.000 mL of water was added into a sealed pressure vessel, and then the recovery rate of water added was examined when the above extraction method had been employed.

**[Table 1]**

| | Amount added [mL] | Volume of recoverd liquid [mL] | Recovery rate [%] |
|---|---|---|---|
| 1 | 8.000 | 7.210 | 90.0 |
| 2 | 8.000 | 7.372 | 92.1 |
| 3 | 8.000 | 7.391 | 92.4 |
| | | Average | 91.5 |

As shown in Table 1, the recovery rate of water added was 91.5% on average of the triplicates, and the solution recovery rate was 90% or more. These are recovery rates equivalent to those of a conventional extraction method such a method using DMSO.

### Example 3 Composition of main components of particulate matter extracted from smoke of heated smoking article

In this example, the composition of the main components of particulate matter extracted from the smoke of heated smoking articles was examined by various methods.

The heated smoking articles used herein were heated smoking articles (type of producing aerosols by transfer of heat from a heat source to an aerosol-forming substrate, iQOS, Philip Morris).

The solventless extraction process (SEP) was performed in the same manner as in Example 1. Twelve CFs were used, and the first to fourth compression-release cycles were performed in the same manner as in Example 1 for four times.

Extraction with isopropanol (IPA) was performed according to 7.9.1 Extraction Procedure of ISO 4387: 2017 (NPL 7).

Extraction with dimethylsulfoxide (DMSO) (low concentration) was performed according to HCI Regime T-501 (Health Canada Official Method T-501 Bacterial Reverse Mutation Assay for Mainstream Tobacco Smoke) (NPL 8).

Continuous extraction refers to the work of extraction from multiple glass fiber filters with the same extract. When the concentration did not reach the predetermined concentration by single extraction, continuous extractions are performed. Continuous extraction with DMSO was performed as follows.

Continuous DMSO extraction was performed such that the concentration of the extracted particulate matter was 200 mg/mL finally. After the shaking extraction and centrifugation of the first CF are completed, the entire amount of the extract liquid is added to a Duran bottle containing another CF, and then subjected to shaking extraction/centrifugation. This operation is repeated for a predetermined number of filters.

The composition of the main components of the extracted particulate matter was analyzed by the method of Cholesta Recommended Method No. 84 (NPL 9). The results are shown in Table 2.

**[Table 2]**

| | IPA extraction [mg/cig.] | DMSO extraction [mg/cig.] Numericalvalue in parentheses is ap concentration when continuous extraction was performed such that the concentration of particulate matter was 200mg/mL. | SEP [mg/cig.] |
|---|---|---|---|
| **PG** | 0.4 | 0.4 | 0.4 |
| G | 4.2 | 4.0 | 4.1 |
| Nicotine | 1.2 | 1.1 (1.6) | 1.2 |
| Water | 24.2 | 26.5 (36.3) | 24.0 |

In Table 2, "PG" denotes propylene glycol and "G" denotes glycerin. The composition of the main components of the particulate matter extracted by SEP is similar to those resulting from IPA extraction or DMSO extraction (low concentration), suggesting that SEP achieves appropriate extraction of main components of the particulate matter in the smoke of the heated smoking article.

### Example 4 Bacterial reverse mutation test and micronucleus test

In this example, the extract liquid obtained in Example 3 and containing particulate matter in the smoke of the heated smoking article extracted by the solventless extraction process (SEP) was used to perform the bacterial reverse mutation test (Ames test) and the micronucleus test (MN test).

The following samples were prepared as test substances for the Ames test and the MN test.
SEP sample: iQOS (1000 mg/mL) (solvent in SEP: PG/G/water) (1.2% addition)
DMSO extraction sample: iQOS (200 mg/mL) (10 continuous extractions with DMSO) (1% addition) (100 mg/mL is the limit for typical extraction with DMSO)

### (1) Ames Test

The Ames test was performed according to "Bacterial reverse mutation test" (OECD / OCDE TG471) of "OECD Guidelines for the Testing of Chemicals" (adopted July 21, 1997). Specifically, the test was performed as follows.

Five test bacterial strains *(Salmonella typhimurium* TA98, TA100, TA1535, TA1537 and TA102) obtained from the National Institute of Health Sciences (Kanagawa, Japan) were used. In the presence and the absence of a metabolic activation system, total particulate matter (TPM) fractions were tested for mutagenicity.

A reaction mixture containing approximately 1 × 10⁹ cells of a bacterial culture, a serially diluted test sample (SEP sample or DMSO extraction sample), and S9 mix containing phenobarbital/5,6-benzoflavone-induced rat liver homogenate or PBS was incubated at 37°C ± 1°C for 20 minutes ± 1 minute.

As a positive control, furylfuramide (FUJIFILM Wako Pure Chemical Corporation, Tokyo, Japan) was used for TA98, TA100, and TA102, sodium azide (FUJIFILM Wako Pure Chemical Corporation, Tokyo, Japan) was used for TA1535, and ICR-191 (Sigma Aldrich; St. Louis, MO, USA) was used for TA1537, in a test without metabolic activation. In the test with metabolic activation, 2-aminoanthracene (FUJIFILM Wako Pure Chemical Corporation, Tokyo, Japan) was used for TA102 and TA1535, and benzopyrene (FUJIFILM Wako Pure Chemical Corporation, Tokyo, Japan) was used for TA98, TA100 and TA1537. Only DMSO was used as a solvent control.

After incubation, the reaction mixture was mixed with thawed top agar and plated on minimum glucose agar plates. The plates were incubated at 37°C ± 1°C for 48-72 hours and revertants per plate were automatically counted. The average number of revertants was calculated from the results of 3 plates. Two or three independent tests were performed using the independently prepared test samples.

It was determined that the sample is mutagenic, when the following conditions were fulfilled: in all bacterial strain + S9 combinations, a revertant increased in a reproducible and dose-dependent manner; and, at one or more concentrations of a test sample, at least 2-fold increase relative to the solvent control was found on TA98, TA100 and TA102, or, at one or more concentrations of a test sample, at least 3-fold increase relative to the solvent control was found on TA1535 and TA1537.

To compare immunogenicity among test samples, JMP version 10.0.2 (SAS Institute Japan, Tokyo, Japan) was used to calculate the slope parameter (i.e., linear coefficient) based on linear regression analysis on the linear portion of the dose curve.

### (2) MN test

The MN test was performed according to "IN VITRO mammalian cell micronucleus test" (OECD/OCDE TG487) of "OECD Guidelines for the Testing of Chemicals" (adopted September 26, 2014). Specifically, the test was performed as follows.

The CHO ± U cell line purchased from Sigma Aldrich (St. Louis, MO, USA) was used for the analysis. Cells were maintained in DMEM supplemented with 10% fetal calf serum (Sigma Aldrich (St. Louis, MO, USA) )at 37°C ± 2°C in a 5% CO₂ incubator.

Total particulate matter (TPM) fractions were analyzed under three treatment schedule types: short-term exposure without metabolic activation, short-term exposure with metabolic activation, and long-term exposure without metabolic activation.

The cell suspension (2 × 10⁴ cells/well) was pre-incubated for 24 hours ± 3 hours before treatment. For short-term exposure, cell cultures were treated with test samples (SEP samples or DMSO extraction samples) for 3 hours ± 15 minutes together with or without S9 mix containing Arcolor 1254-induced rat liver homogenate. After removal of the test samples, cells were incubated for 21 hours ± 1 hour. For long-term exposure, cells were incubated for 24 hours ± 1 hour in the absence of a metabolic activation system. As a positive control, mitomycin C (Sigma Aldrich (St. Louis, MO, USA) was applied in the absence of metabolic activation and cyclophosphamide (Sigma Aldrich (St. Louis, MO, USA) was applied in the presence of metabolic activation. DMSO alone was used as a solvent control.

Cells were fixed with a solution of glacial acetic acid/methanol (1:99, v/v), washed with a solution of ethanol/water (70%, v/v), and then stained with a DAPI solution and a CellMask orange solution (Thermo Fisher Scientific Inc.; Waltham, MA, USA).

All wells containing stained cells were scanned by Array Scan (manufactured by Thermo Fisher Scientific Inc.). The number of micronucleated cells per 2000 cells (1000 cells/slide, double culture) was counted, and the micronucleus cell frequency (% MN) was calculated. The experiment was performed twice independently.

The genotoxicity of each sample was evaluated according to a method described in Matsushima et al., 1999, Environ. Pollut. 64, 121-132 (NPL 10). Cochrane-Armitage binomial test was used to examine the dose dependence of MN frequency. Fisher's exact test was used to assess whether the MN frequency was significantly increased at one or more concentrations of the test sample relative to the solvent control in the parallel runs.

It was determined that a test sample is genotoxic, when the results of both statistical tests were significant in two independent experiments and reproducible. For comparison of genotoxicity between test samples, logistic regression analysis was performed on the data up to the concentration at which the MN frequency reached the maximum value. The gradient parameter of logistic counting was identified as genotoxic activity. Data analysis was performed using JMP version 10.0.2 (SAS Institute Japan, Tokyo, Japan).

### (3) Results

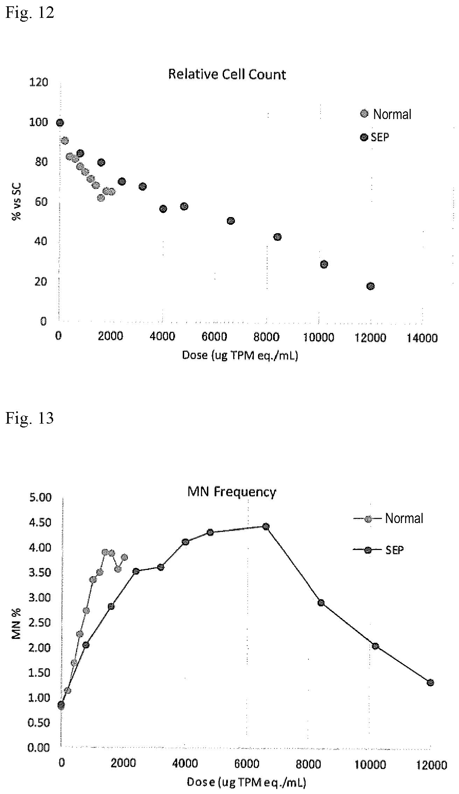

Fig. 12 shows the results of the Ames test. The horizontal axis plots the added doses of samples (µg total particulate matter (TPM) eq./mL), and the vertical axis plots the ratio (%) of the number of viable cells (cells that have undergone cell reversion mutation) after the addition of a sample to the number of viable cells before the addition of the sample.

Fig. 13 shows the results of the MN test. The horizontal axis plots the added doses of samples (µg total particulate matter (TPM) eq./mL), and the vertical axis plots the frequency (%) of the occurrence of micronuclei (MN) (MN induction rate).

The item 28 of OECD/OCDE TG487 of the OECD guidelines for MN test states, "The highest concentration should aim to achieve 55.5 ± 5% cytotoxicity." " Where cytotoxicity occurs, the test concentrations selected should cover a range from that producing 55.5 ± 5% cytotoxicity and including concentrations at which there is moderate and little or no cytotoxicity": however, when a DMSO extraction sample was used, exposure was possible to achieve only up to about 40% cytotoxicity (Fig. 12, normal). This is because the dose higher than 2000 µg TPMeq./ml exceeds the added dose of 2%, at which the cytotoxicity of a DMSO solvent can be mixed therein. When the DMSO extraction sample was used, any downturn of MN induction was not confirmed (Fig. 13, normal). All of the results are insufficient although these are the MN test results.

On the other hand, when the SEP samples (samples obtained by SEP) were used, exposure was possible to achieve up to almost 100% cytotoxicity (Fig. 12, SEP). A peak and downturn of MN induction was confirmed (Fig. 13, SEP, dose higher than the dose of 6000 µg TPM eq./ml). The item 28 of OECD/OCDE TG487 of the OECD guidelines for the MN test states, "The highest concentration should aim to achieve 55.5 ± 5% cytotoxicity." " Where cytotoxicity occurs, the test concentrations selected should cover a range from that producing 55.5 ± 5% cytotoxicity and including concentrations at which there is moderate and little or no cytotoxicity." It was found that the test according to the OECD guidelines can be performed using the SEP samples.

### Example 5 Neutral red uptake activity test

### (1) Materials etc.

### (Test samples)

Cigarette Samples: Kentucky Reference Cigarette 3R4F are cigarettes with filters, which have been provided by the Tobacco and Health Research Institute, the University of Kentucky, for research purposes (partially modified from Japanese Translation of PCT International Application Publication No. 2008-544938).

Heated smoking articles:
Sample A = a type of producing aerosols by the transfer of heat from a heat source inserted inside a heated smoking article to an aerosol-forming substrate, a heated smoking article (trade name: Marlboro for iQOS REGULAR (trademark)), manufactured by Philip Morris Products Societe Anonym was used with a dedicated heating type smoking device (trade name: iQOS2.4 (registered trademark)) manufactured by Philip Morris Products Societe Anonym.
Sample B = a type of producing aerosols by the transfer of heat from a heat source disposed on the outer periphery of a heated smoking article to an aerosol-forming substrate was used with a dedicated heating type smoking device.
Sample C = a heated smoking article, Mevius (registered trademark) Regular for Ploom TECH (manufactured by Japan Tobacco Inc.) was used with an enclosed cartomizer and a dedicated smoking device (Ploom (registered trademark) Tech). The samples were stored at 4°C until just before the start of the next humidity control/conditioning.

(Humidity control/conditioning) All the samples were subjected to humidity control/conditioning according to the standard method ISO3402: 1999 (NPL 5). (Inhalation conditions) Standard method HCI
(End of inhalation) Standard method ISO4387: 2017 (NPL 7)

### (Smoke/aerosol collection)

ACM (aerosol collected mass) (the amount of aerosols produced from non-combustion type cigarette) collected by SEP was weighed with an electronic balance together with a Cambridge filter, and the tare of the Cambridge filter was subtracted to obtain the ACM weight.

### (Extraction of smoke and aerosol)

In the conventional method, the total particulate matter/collected aerosol matter of smoke collected in the CFP were extracted by adding DMSO so that the concentration was 100 mg/mL according to the conventional method.

### (Preparation of in vitro test sample)

The DMSO extraction solution of smoke/aerosols extracted by a conventional method was adjusted by adding a DMSO solvent so that the concentration was 100 mg/mL in terms of the TPM/ACM weight, which was weighed in advance in this example.

The smoke/aerosol solution extracted by SEP was used as an *in vitro* test sample without any adjustment.

### (2) Neutral red uptake activity test

The neutral red uptake activity test was performed using CHO-K1 cells following the operating procedure according to Health Canada Official Method T-502, Neutral Red Uptake Assay for Mainstream Tobacco Smoke (Health Canada, 2004c) (NPL 11).

Cells were maintained in a 5% CO₂ incubator at 37°C ± 2°C in Ham's F12 nutrient mixed medium (Thermo Fisher Scientific Inc.; Waltham, MA, USA) supplemented with 10% FBS (Sigma Aldrich; St. Louis, MO, USA).

The CHO-K1 cell suspension (1.0 × 10⁴ cells/well) was pre-incubated in 96-well microtiter plates for 24 hours ± 2 hours. Cells were treated with the total particulate matter (TPM) fraction for 24 hours. Sodium dodecyl sulfate was used as a positive control. Only DMSO or CMF-PBS (calcium-magnesium free-PBS) was used as a solvent control for the TPM or GVP test, respectively.

In one experiment, three 96-well plates were analyzed per test sample. Cells treated as described above were incubated for 3 hours in medium containing 18 µg/mL neutral red dye. In pairs, cells were fixed with 1% formalin for 1-2 minutes. After removing a fixative, the neutral red dye taken up by living cells was extracted by adding 50% ethanol containing 1% (v/v) acetic acid, and the absorbance at 540 nm was measured using a microplate reader. Three independent experiments were performed.

The cytotoxicity at each treatment level was expressed in terms of absorbance relative to that of a solvent control of the parallel runs. A non-linear regression analysis was performed on the relationship between relative absorbance and concentration based on the least squares method using the logistic coefficient, in order to calculate IC50 values for comparison of cytotoxic activity between test samples. The IC50 value was calculated by inverse estimation of the effective concentration at the time when the absorbance was reduced to 50% of the value of the solvent control in the parallel runs. Calculations were performed using JMP version 10.0.2 (SAS Institute Japan, Tokyo, Japan).

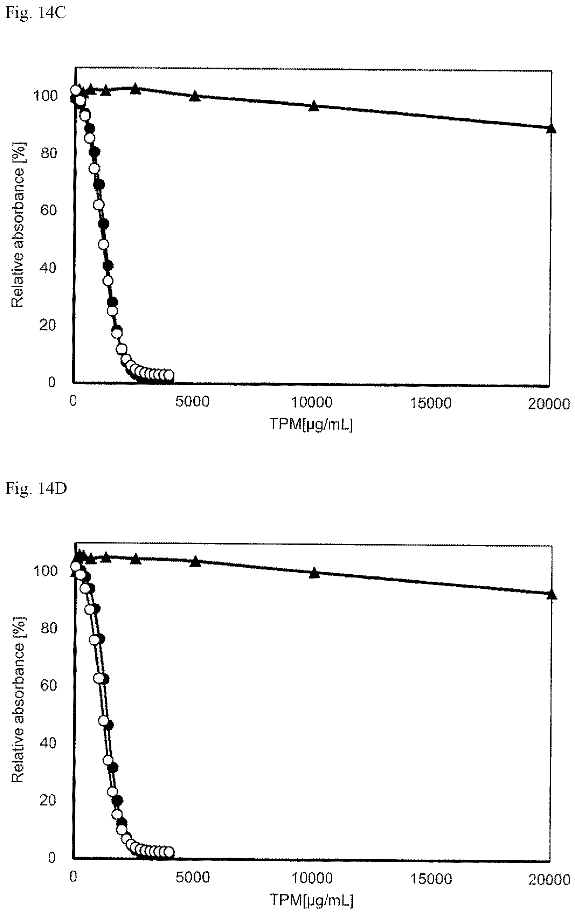

The results are shown in Fig. 14. In Fig. 14, the horizontal axis plots the doses of samples added, and the vertical axis plots the percentage of the neutral red uptake of the cells exposed to the sample relative to the neutral red uptake of the cells to which no sample was added. The test by conventional method (DMSO) was performed once (Fig. 14A) and the test by SEP was performed in triplicate (three independent tests in Figs. 14B to 14D).

When the DMSO extraction sample was used for the cigarette sample, exposure was possible to achieve such an extent that the neutral red uptake activity almost disappeared. On the other hand, for the heated smoking article samples, specifically, both the sample A, i.e., heated smoking article iQOS, and the sample B, i.e., heated smoking article B, exposure was possible to achieve only about 70% of the neutral red uptake activity (Fig. 14A). Regarding the heated smoking article PloomTECH, the neutral red uptake activity did not change (Fig. 14A). In any of the heated smoking article samples, the technical limit for adjusting the DMSO extraction sample was 1000 µg/ml of TPM.

On the other hand, when the SEP samples were used for the sample A, i.e., heated smoking article iQOS, and the sample B, i.e., heated smoking article B, exposure was possible for both samples to achieve to such an extent that the neutral red uptake activity disappeared (Fig. 14B, Fig. 14C, and Fig. 14D). For sample C, i.e., heated smoking article PloomTECH, exposure was possible to achieve about 90% neutral red uptake activity (Figs. 14B, C, and D). This is because SEP is a method for extracting the undiluted solution of aerosols produced by a heated smoking article on extraction principles, but even the undiluted solution of the PloomTECH sample has an upper limit of 20000 µg/ml of TPM. Specifically, it was demonstrated that the neutral red uptake activity test can be completed using the aerosol component of the heated smoking article as a sample obtained by SEP. As is clear from Fig. 14, comparative tests were successfully performed on various types of heated smoking articles including heated smoking articles in which aerosols are produced by the transfer of heat from a heat source inserted inside the heated smoking article to an aerosol-forming substrate, heated smoking articles in which aerosols are produced by the transfer of heat from a heat source disposed on the outer periphery of the heated smoking article to an aerosol-forming substrate, and infused heated smoking articles.

### REFERENCE SIGNS LIST

1a to If Extraction container
2a to 2f First squeezing member
3a to 3f Second squeezing member
4, 4e, 4f, 4g First squeezing surface
5, 5e, 5f, 5g Second squeezing surface
6, 6g Recess
7 Stacked filter members
8, 8g Accommodation area
9 Groove
10, 10g, (40, 45), 54 Reservoir
11 Tip portion of second squeezing member
12 O-ring
13 Thin tube
14 Trumpet-shaped opening
20 Housing
21 Lid portion
22 Screw through hole
23 Screw rod
24 Handle
25 Bearing portion
26 Overhanging bottom plate of bearing portion
30, 31, 32 Bolt
40 First cavity portion (reservoir, tube connector)
45 Second cavity portion (reservoir, tube connector)
41, 46 Large-diameter portion
42, 47 Medium-diameter portion
43, 48 Small-diameter portion
44, 49 Escape port of circulation tube
50 Circulation tube
51 One end of circulation tube 50
52 Other end of circulation tube 50
53 Peristaltic pump
55 Annular Groove
60 Sieve member
61 Tip portion
62 Proximal space

## Claims

1. A method for extracting particulate matter from the smoke of a heated smoking article, comprising:
(1) collecting particulate matter in the smoke of a heated smoking article in two or more filters capable of capturing the particulate matter,
(2) stacking the two or more filters, and
(3) squeezing the stacked filters to extract components adhered to the filters.

2. The method according to claim 1, wherein step (3) is performed by
(3-i) compressing and releasing the stacked filters,
(3-ii) compressing the stacked filters to extract components adhered to the filters, pouring the extract liquid to the stacked filters, and then compressing the filters again, or
(3-iii) performing (3-i) and (3-ii) in combination.

3. The method according to claim 2, wherein step (3-i) of compressing and releasing the stacked filters is repeated two or more times.

4. The method according to claim 2, wherein the filters and the extract liquid are shaken together during steps (3-i) and/or (3-ii).

5. The method according to claim 2, wherein step (3-ii) of compressing the stacked filters to extract components adhered to the filters, pouring back the extract liquid to the stacked filters, and then compressing the filters again is repeated two or more times.

6. The method according to any one of claims 1 to 5, wherein step (3) is performed on the filters accommodated in an extraction container having two squeezing surfaces.

7. The method according to any one of claims 1 to 6, wherein in step (3), the extract liquid obtained by squeezing is made to flow or circulate.

8. The method according to any one of claims 1 to 7, wherein three or more filters are used.

9. The method according to any one of claims 1 to 8, wherein 10 to 15 filters are used.

10. The method according to any one of claims 1 to 9, wherein step (3) is repeated until the thickness of the stacked filters becomes 1/3 or less than the thickness before performing step (3).

11. The method according to any one of claims 1 to 10, wherein no solvent is used in step (3).

12. The method according to any one of claims 1 to 10, wherein in step (3), an amphipathic solvent is added in an amount of more than 0% (weight/volume) and 400% (weight/volume) or less with respect to the filter volume.

13. The method according to any one of claims 1 to 12, wherein compression is performed at 10 MPa or less.

14. The method according to any one of claims 1 to 13, wherein the filters are glass fiber filters.

15. The method according to any one of claims 1 to 14, wherein the filters have a particle collection efficiency of 99% or more for collecting particles having a particle size of 0.3 µm at a rated air volume.

16. The method according to any one of claims 1 to 15, further comprising
(4) centrifuging the filters to obtain a separated liquid; and
(5) mixing the separated liquid with the extract liquid obtained in step (3).

17. An extraction container, comprising
an accommodation area capable of accommodating two or more filters in a stacked state,
one or more squeezing members for squeezing the filters accommodated in the accommodation area in a stacked state; and
a reservoir communicating with the accommodation area.

18. The extraction container according to claim 17, wherein the shape of a squeezing surface for squeezing the filters with the squeezing member is a flat surface, a conical slope, or a convex or concave curved surface.

19. The extraction container according to claim 17 or 18, wherein there are two squeezing surfaces for squeezing the filters with the squeezing member.

20. The extraction container according to claim 19, wherein the two squeezing surfaces rotate and slide relative to each other.

21. The extraction container according to any one of claims 17 to 20, wherein the reservoir is positioned at least either above or below the accommodation area.

22. The extraction container according to any one of claims 17 to 20, wherein the reservoir is provided on the outer periphery of the accommodation area.

23. The extraction container according to any one of claims 17 to 21, wherein the reservoir communicates with the accommodation area through a thin tube that penetrates at least one of the squeezing surfaces.

24. The extraction container according to any one of claims 17 to 23, wherein a groove is provided on the squeezing surface of the squeezing member.

25. The extraction container according to any one of claims 17 to 24, having the two squeezing members, wherein
the two squeezing members are movable with the squeezing surfaces thereof facing each other, and
the space between the squeezing surfaces facing each other of the two squeezing members defines the accommodation area.

26. The extraction container according to claim 25, wherein
one of the two squeezing members has a recess, the inner bottom surface of the recess serves as a squeezing surface,
the other squeezing member of the two squeezing members is liquid-tightly slidable in the recess with the squeezing surface thereof facing the inner bottom surface.

27. The extraction container according to any one of claims 17 to 20 comprises:
a syringe-type cylinder member,
a sieve member having pores and disposed so as to partition the hollow space of the cylinder member into a proximal space and a distal space, and
a piston member liquid-tightly slidable in the proximal space of the cylinder member, wherein
the sieve member and the piston member serve as the squeezing members, and the inner surfaces thereof facing each other serve as squeezing surfaces,
at least a part of the proximal space between the squeezing surface of the piston member and the squeezing surface of the sieve member defines the accommodation area, and
the distal space defines the reservoir.

28. The extraction container according to any one of claims 17 to 27, wherein the extract liquid recovered in the reservoir is made to flow or circulate.

29. The extraction container according to any one of claims 17 to 28 for use in the method according to any one of claims 1 to 16.

30. An extract liquid containing particulate matter in the smoke of a heated smoking article, obtained by the method according to any one of claims 1 to 16.

31. The extract liquid according to claim 30, containing no solvent.

32. The extract liquid according to claim 30 or 31 for use in an *in vitro* toxicity test.

33. The extract liquid according to claim 32, wherein the *in vitro* toxicity test is a micronucleus test or an Ames test.

34. An *in vitro* toxicity test method, including using an extract liquid containing particulate matter in the smoke of a heated smoking article obtained by the method according to any one of claims 1 to 16.

## Amended claims

### Amended claims under Art. 19.1 PCT

1. A method for extracting particulate matter from the smoke of a heated smoking article, comprising:
(1) collecting particulate matter in the smoke of a heated smoking article in two or more filters capable of capturing the particulate matter,
(2) stacking the two or more filters, and
(3) squeezing the stacked filters to extract components adhered to the filters.

2. The method according to claim 1, wherein step (3) is performed by
(3-i) compressing and releasing the stacked filters,
(3-ii) compressing the stacked filters to extract components adhered to the filters, pouring the extract liquid to the stacked filters, and then compressing the filters again, or
(3-iii) performing (3-i) and (3-ii) in combination.

3. The method according to claim 2, wherein step (3-i) of compressing and releasing the stacked filters is repeated two or more times.

4. The method according to claim 2, wherein the filters and the extract liquid are shaken together during steps (3-i) and/or (3-ii).

5. The method according to claim 2, wherein step (3-ii) of compressing the stacked filters to extract components adhered to the filters, pouring back the extract liquid to the stacked filters, and then compressing the filters again is repeated two or more times.

6. The method according to any one of claims 1 to 5, wherein step (3) is performed on the filters accommodated in an extraction container having two squeezing surfaces.

7. The method according to any one of claims 1 to 6, wherein in step (3), the extract liquid obtained by squeezing is made to flow or circulate.

8. The method according to any one of claims 1 to 7, wherein three or more filters are used.

9. The method according to any one of claims 1 to 8, wherein 10 to 15 filters are used.

10. The method according to any one of claims 1 to 9, wherein step (3) is repeated until the thickness of the stacked filters becomes 1/3 or less than the thickness before performing step (3).

11. The method according to any one of claims 1 to 10, wherein no solvent is used in step (3).

12. The method according to any one of claims 1 to 10, wherein in step (3), an amphipathic solvent is added in an amount of more than 0% (weight/volume) and 400% (weight/volume) or less with respect to the filter volume.

13. The method according to any one of claims 1 to 12, wherein compression is performed at 10 MPa or less.

14. The method according to any one of claims 1 to 13, wherein the filters are glass fiber filters.

15. The method according to any one of claims 1 to 14, wherein the filters have a particle collection efficiency of 99% or more for collecting particles having a particle size of 0.3 µm at a rated air volume.

16. The method according to any one of claims 1 to 15, further comprising
(4) centrifuging the filters to obtain a separated liquid; and
(5) mixing the separated liquid with the extract liquid obtained in step (3).

17. (deleted)

18. (deleted)

19. (deleted)

20. (deleted)

21. (deleted)

22. (amended) An extraction container, comprising
an accommodation area capable of accommodating two or more filters in a stacked state,
one or more squeezing members for squeezing the filters accommodated in the accommodation area in a stacked state; and
a reservoir communicating with the accommodation area,
wherein the reservoir is provided on the outer periphery of the accommodation area.

23. (deleted)

24. (deleted)

25. (deleted)

26. (deleted)

27. (deleted)

28. (deleted)

29. (deleted)

30. An extract liquid containing particulate matter in the smoke of a heated smoking article, obtained by the method according to any one of claims 1 to 16.

31. The extract liquid according to claim 30, containing no solvent.

32. The extract liquid according to claim 30 or 31 for use in an *in vitro* toxicity test.

33. The extract liquid according to claim 32, wherein the *in vitro* toxicity test is a micronucleus test or an Ames test.

34. An *in vitro* toxicity test method, including using an extract liquid containing particulate matter in the smoke of a heated smoking article obtained by the method according to any one of claims 1 to 16.
